# EUROPEAN PATENT SPECIFICATION

(11) **EP 2 730 209 B1**
(45) Date of publication and mention of the grant of the patent: **12.08.2020**
(21) Application number: 12807713.8
(22) Date of filing: 12.06.2012
(51) Int. Cl.: A61B 1/00, A61B 1/04, A61B 1/06, A61B 5/1455, H04N 7/18

(54) **ENDOSCOPE SYSTEM**
ENDOSKOPSYSTEM
SYSTÈME D'ENDOSCOPE

(30) Priority: 06.07.2011 JP 2011149994
(43) Date of publication of application: 14.05.2014
(73) Proprietor: FUJIFILM Corporation, Tokyo 106-8620 (JP)
(72) Inventor: SAITO, Takaaki, Ashigarakami-gun Kanagawa 258-8538 (JP); YAMAGUCHI, Hiroshi, Ashigarakami-gun Kanagawa 258-8538 (JP); IIDA, Takayuki, Ashigarakami-gun Kanagawa 258-8538 (JP)
(74) Representative: Klunker IP Patentanwälte PartG mbB
(86) International application number: PCT/JP2012/064974
(87) International publication number: WO 2013/005533

(56) References cited:
- EP-A1- 2 305 094
- JP-A- 10 108 073
- JP-A- 2003 508 735
- JP-A- 2005 198 794
- JP-A- 2006 101 975
- JP-A- 2010 279 454
- JP-A- 2011 092 690
- US-A- 5 408 998

## Description

### TECHNICAL FIELD

The present invention relates to an endoscope system that observes the inside of a body cavity using special light having a specific wavelength, a processor device of the endoscope system, and an image display method.

### BACKGROUND ART

In a current medical field, a cancer diagnosis using an endoscope is widely carried out. In this endoscopic cancer diagnosis, an insert section of the endoscope is introduced into a human body cavity. While illumination light having a predetermined wavelength is applied from a distal portion of the insert section to an observation object, the observation object is imaged by an imaging device provided at the distal portion to obtain an image in which various types of biological information appearing in the observation object are reflected. For example, according to Japanese Patent No. 3559755, an image in which superficial blood vessels and a superficial fine structure are emphasized, though which are inconspicuous under broad band illumination light such as white light, is obtained by using narrow band light having a specific wavelength as the illumination light. Performing a diagnosis using such an image, which clearly shows the superficial blood vessels and the superficial fine structure, makes it possible not only to distinguish a cancer but also to estimate the stage of the cancer.

Also, according to Japanese Patent No. 2648494, an oxygen saturation level of hemoglobin in blood is imaged by using light having a wavelength band in which a light absorption coefficient is different between oxyhemoglobin and deoxyhemoglobin as the illumination light. For example, a cancer having a certain extent shows a hypoxic state at its center, while showing a hyperoxic state at its periphery. Thus, using an image of the oxygen saturation level, as described above, facilitates grasping the condition of a cancer intuitively.

EP 2 305 094A1 forms the basis for the preambles of claims 1 and discloses an endoscope system having a lighting section and an image signal obtaining section as defined by the first and second feature of claim 1, respectively. According to this reference, there are provided first to third narrow band signals. The luminance ratios between the first and third narrow band signals and the second and third narrow band signals are calculated. Vessel depth information and oxygen saturation information are obtained at the same time based on correlations between said luminance ratios on the one hand and vessel depth and oxygen saturation, on the other hand, which correlations are stored in advance. That is to say, both the first image signal and the second image signal are necessary to produce either the first object image or the second object image. US 5 408 998 A discloses an endoscope system used to illustrate blood oxygen saturation and/or tissue perfusion. Three wavelength bands are used to obtain three image signal components. Three wavelength bands of illumination light are obtained by a broad band light source and an optical filter wheel.

### SUMMARY OF THE INVENTION

### Problems to be Solved by the Invention

In the case of performing the cancer diagnosis based on the superficial fine blood vessels and the like shown with emphasis, as described in the Japanese Patent No. 3559755, it is required to have knowledge of a blood vessel pattern and the like specific to a cancer in advance. Also, to estimate the stage of the cancer from the blood vessel pattern, considerable knowledge and experience are required. On the other hand, in the case of performing the cancer diagnosis using the image of the oxygen saturation level, as described in the Japanese Patent No. 2648494, a cancer is easily distinguishable. However, as for the detailed cancer diagnosis of the stage of the cancer and the like, the information of a blood vessel shape including a pattern of the superficial fine blood vessels is required in addition to the information of the oxygen saturation level.

The present invention aims at providing an endoscope system that can grasp both blood vessel shape information of superficial fine blood vessels and the like and an oxygen saturation level of hemoglobin in blood, which are used for diagnosing a lesion such as a cancer, a processor device of the endoscope system, and an image display method.

### Means for Solving the Problems

To achieve the above object, an endoscope system according to the present invention includes the features of claim 1.

The image signal obtaining section preferably includes a positioning unit for performing positioning of an object image between the first and second image signals.

It is preferable that the image signal obtaining section further includes a structure enhancing unit for applying a structure enhancing process to the first and second image signals to enhance a structure of the observation object, and the positioning unit performs positioning of the object image between the first and second image signals after being subjected to the structure enhancing process. The structure of the observation object preferably includes a blood vessel structure.

The lighting section may include a broad band light source for emitting broad band light in a broad wavelength band and a rotary filter for sequentially transmitting the first and second illumination light out of the broad band light. The lighting section may include a plurality of semiconductor light sources for emitting the first and second illumination light.

The first illumination light contains at least blue narrow band light having a wavelength band in a blue region and green narrow band light having a wavelength band in a green region. The second illumination light contains at least narrow band light having two non-isosbestic wavelengths at which a magnitude relation of a light absorption coefficient between the oxyhemoglobin and the deoxyhemoglobin differs from each other, and narrow band light having an isosbestic wavelength at which the oxyhemoglobin and the deoxyhemoglobin have the same light absorption coefficient. The first object image is a blood vessel enhanced image in which a superficial blood vessel and a middle to deep-layer blood vessel are enhanced. The second object image is an oxygen saturation image, which images an oxygen saturation level of hemoglobin in blood.

It is preferable that the blue narrow band light is in a wavelength band of 410±10 nm, the narrow band light having the two non-isosbestic wavelengths is in wavelength bands of 440±10 nm and 470±10 nm, and the green narrow band light and the narrow band light of the isosbestic wavelength are in a wavelength band of 540±10 nm.

It is preferable that the second illumination light further includes narrow band light in a wavelength band of 650±10 nm and narrow band light in a wavelength band of 910±10 nm, as the narrow band light having the non-isosbestic wavelengths.

### Effect of the Invention

According to the present invention, the first object image and the second object image are obtained. The first object image is produced with irradiation with the first illumination light in a wavelength band having a high light absorption coefficient of hemoglobin, out of the light in a wavelength band penetrating to a specific depth in living body tissue. The second object image is produced with irradiation with the second illumination light in a wavelength band in which oxyhemoglobin and deoxyhemoglobin have different light absorption coefficients. By displaying the first and second object images on the display section, it is possible to grasp information about the shape of a blood vessel including superficial capillary vessels and the like from the first object image, and grasp the oxygen saturation level of hemoglobin in blood from the second object image.

In the case of sequentially applying at least the first illumination light and the second illumination light, the positioning of the object image is performed between the first image signal obtained by imaging the observation object under irradiation with the first illumination light and the second image signal obtained by imaging the observation object under irradiation with the second illumination light. Therefore, it is possible to obtain the first and second object images of high quality without occurrence of artifact and the like.

### BRIEF DESCRIPTION OF DRAWINGS

Fig. 1 is a perspective view of an endoscope system;
Fig. 2 is a schematic view showing the internal structure of the endoscope system according to a first embodiment;
Fig. 3 is a plan view of a rotary filter for normal observation;
Fig. 4 is a plan view of a rotary filter for special observation;
Fig. 5 is a diagram for explaining the operation of a CCD in a normal observation mode;
Fig. 6 is a diagram for explaining the operation of the CCD in a special observation mode;
Fig. 7 is a diagram for explaining the positioning among enhanced first to fourth narrow band images;
Fig. 8 is a diagram in which a blood vessel enhanced image and an oxygen saturation image are displayed side to side on a monitor;
Fig. 9 is a graph showing a light absorption coefficient of hemoglobin and the light amount distributions of first and fourth narrow band light;
Fig. 10 is a graph showing a light absorption coefficient of oxyhemoglobin and deoxyhemoglobin;
Fig. 11 is a flowchart for explaining an operation flow in the special observation mode;
Fig. 12 is a plan view showing a rotary filter for special observation different from that of Fig. 4; and
Fig. 13 is a schematic view showing the internal structure of the endoscope system according to a second embodiment.

### DESCRIPTION OF INVENTION

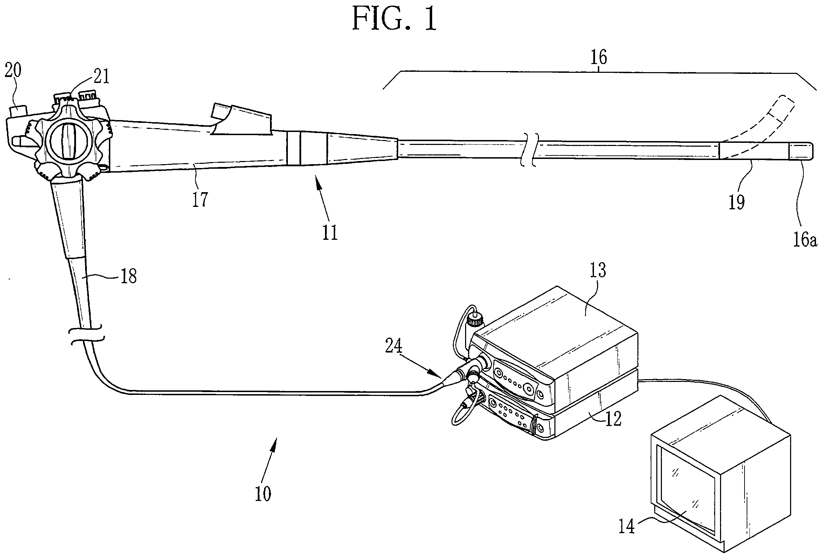

As shown in Fig. 1, an endoscope system 10 according to a first embodiment is constituted of an electronic endoscope 11 for imaging the inside of a human body cavity, a processor device 12 for producing an image based on a signal obtained by the imaging, a light source device 13 for supplying light for illuminating the inside of the body cavity, and a monitor 14 for displaying the image produced by the processor device 12.

This endoscope system 10 is switchable between a normal observation mode using light having a wavelength band extending from blue to red as illumination light of an observation object and a special observation mode using narrow band light having a wavelength limited within a specific band as the illumination light. The switching between these modes is performed with a mode switch SW 20 provided on the electronic endoscope 11.

The electronic endoscope 11 includes a flexible insert section 16 to be introduced into the body cavity, a handling section 17 provided on a proximal end portion of the insert section 16, and a universal cord 18 for connecting the handling section 17 to the processor device 12 and to the light source device 13.

The insert section 16 has at its distal end a bending portion 19 that is composed of a train of joint pieces. The bending portion 19 flexibly bends up and down and from side to side in response to an operation of an angle knob 21 provided on the handling section 17. The bending portion 19 is provided with a distal end portion 16a, which contains an optical system and the like used for imaging the inside of the body cavity. The distal end portion 16a is aimed at a desired direction by a bending operation of the bending portion 19.

A connector 24 is attached to the universal cord 18 on the side of the processor device 12 and the light source device 13. The connector 24 is a complex type connector including a communication connector and a light source connector. The electronic endoscope 11 is detachably connected to the processor device 12 and the light source device 13 through this connector 24.

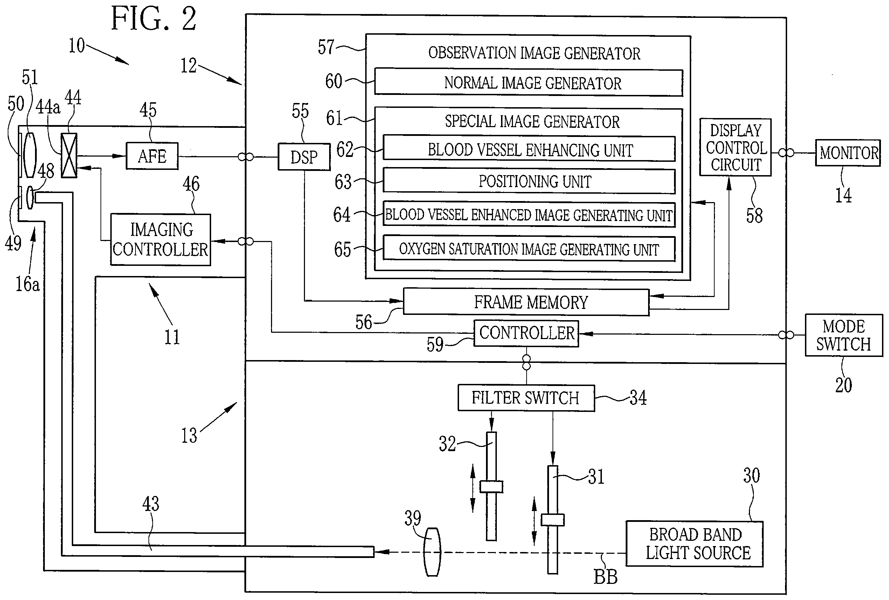

As shown in Fig. 2, the light source device 13 is provided with a broad band light source 30, a rotary filter 31 for normal observation, a rotary filter 32 for special observation, a filter switch 34, and a condenser lens 39. The broad band light source 30 is a xenon lamp, a white LED, a micro white light source, or the like. The broad band light source 30 emits broad band light (white light) BB having a wavelength extending from the blue region to the red region (400 to 700 µm).

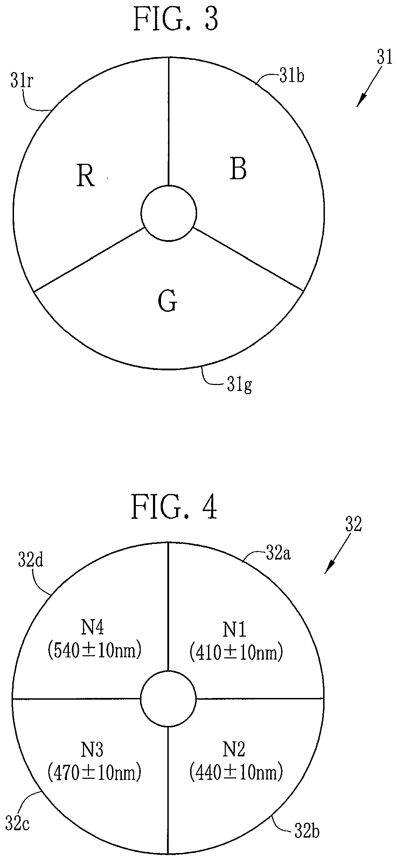

In the normal observation mode, the rotary filter 31 for normal observation is set in an optical path of the broad band light source 30 by the filter switch 34. As shown in Fig. 3, the rotary filter 31 for special observation is composed of a blue light transmitting region 31b for transmitting blue light B in the blue region out of the broad band light BB from the broad band light source 30, a green light transmitting region 31g for transmitting green light G in the green region out of the broad band light BB, and a red light transmitting region 31r for transmitting red light R in the red region out of the broad band light BB, which are disposed in a circumferential direction. Thus, by a rotation of the rotary filter 31 for normal observation, the blue light B, the green light G, and the red light R are taken out of the broad band light BB in a sequential manner. These three colors of light are incident upon a light guide 43 through the condenser lens 39.

In the special observation mode, the rotary filter 32 for special observation is set in the optical path of the broad band light source 30 by the filter switch 34. As shown in Fig. 4, the rotary filter 32 for normal observation is composed of a first narrow band light transmitting region 32a for transmitting first narrow band light N1 in a wavelength band of 410±10 nm out of the broad band light BB from the broad band light source 30, a second narrow band light transmitting region 32b for transmitting second narrow band light N2 in a wavelength band of 440±10 nm out of the broad band light BB, a third narrow band light transmitting region 32c for transmitting third narrow band light N3 in a wavelength band of 470±10 nm out of the broad band light BB, and a fourth narrow band light transmitting region 32d for transmitting fourth narrow band light N4 in a wavelength of 540± nm out of the broad band light BB, which are disposed in a circumferential direction. Thus, by a rotation of the rotary filter 32 for special observation, the first to fourth narrow band light N1 to N4 are taken out of the broad band light BB in a sequential manner. The first to fourth narrow band light N1 to N4 is incident upon the light guide 43 through the condenser lens 39.

As shown in Fig. 2, the electronic endoscope 11 is provided with the light guide 43, a CCD 44, an analog front end circuit (AFE) 45, and an imaging controller 46. The light guide 43 is composed of a large-diameter optical fiber, a bundle of fibers, or the like. The light extracted by the rotary filter 31 for normal observation or the rotary filter 32 for special observation is incident upon an incident end of the light guide 43. On the other hand, an exit end of the light guide 43 is directed to a lighting lens 48 contained in the distal portion 16a, so the light led through the light guide 43 is applied to the observation object. The light reflected from the observation object is incident upon the CCD 44 through an imaging window 50 and a condenser lens 51 contained in the distal portion 16a. Note that, a CMOS imaging device or the like may be used instead of the CCD.

The CCD 44 receives the incident light from the condenser lens 51 at its imaging surface 44a, and accumulates signal charge that is obtained by photoelectric conversion of the received light. The accumulated signal charge is read out as an imaging signal. The read imaging signal is transmitted to the AFE 45. The AFE 45 includes a correlated double sampling circuit (CDS) for applying a correlated double sampling process to the imaging signal from the CCD 44, an automatic gain control circuit (AGC) for amplifying the imaging signal after noise reduction by the CDS, and an analog-to-digital converter (A/D) for converting the imaging signal amplified by the AGC into a digital imaging signal having a predetermined bit number and inputting the digital imaging signal to the processor device 12 (none is shown in the drawing) . Note that, a monochrome CCD, which does not have a color separation filter (for example, an RGB filter) is used as the CCD 44.

The imaging controller 46 is connected to a controller 59 of the processor device 12, and transmits a drive signal to the CCD 44 upon a command from the controller 59. The CCD 44 outputs the imaging signal to the AFE 45 at a predetermined frame rate based on the drive signal from the imaging controller 46.

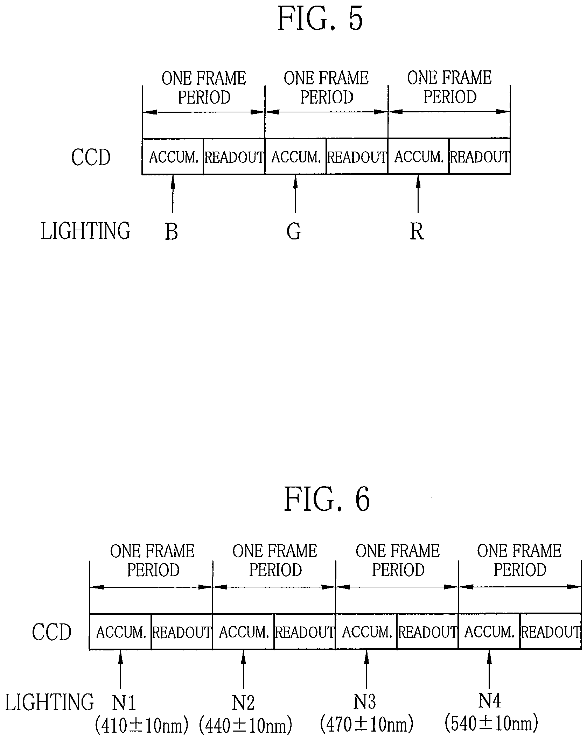

The imaging controller 46 performs different control operations between in the normal observation mode and in the special observation mode. In the normal observation mode, as shown in Fig. 5, two steps i.e. a step of accumulating signal charge produced by photoelectric conversion of the blue light B and a step of reading out the accumulated signal charge as a blue imaging signal are performed in one frame period. In the next one frame period, two steps i.e. a step of accumulating signal charge produced by photoelectric conversion of the green light G and a step of reading out the accumulated signal charge as a green imaging signal are performed. In further next one frame period, two steps i.e. a step of accumulating signal charge produced by photoelectric conversion of the red light R and a step of reading out the accumulated signal charge as a red imaging signal are performed. The blue imaging signal, the green imaging signal, and the red imaging signal read out by the steps in the three frame periods are transmitted to the processor device 12.

On the other hand, in the special observation mode, as shown in Fig. 6 , two steps i.e. a step of accumulating signal charge produced by photoelectric conversion of the first narrow band light N1 and a step of reading out the accumulated signal charge as a first narrow band imaging signal are performed in one frame period. In the next one frame period, two steps i.e. a step of accumulating signal charge produced by photoelectric conversion of the second narrow band light N2 and a step of reading out the accumulated signal charge as a second narrow band imaging signal are performed. In further next one frame period, two steps i.e. a step of accumulating signal charge produced by photoelectric conversion of the third narrow band light N3 and a step of reading out the accumulated signal charge as a third narrow band imaging signal are performed. In further next one frame period, two steps i.e. a step of accumulating signal charge produced by photoelectric conversion of the fourth narrow band light N4 and a step of reading out the accumulated signal charge as a fourth narrow band imaging signal are performed. The first to fourth narrow band imaging signals read out by the steps in the four frame periods are transmitted to the processor device 12.

As shown in Fig. 2, the processor device 12 is provided with a digital signal processor (DSP) 55, a frame memory 56, an observation image generator 57, and a display control circuit 58, and the controller 59 controls these parts. The DSP 55 applies color separation, color interpolation, white balance adjustment, gamma correction, and the like to the imaging signal transmitted from the electronic endoscope 11, and produces an image corresponding to each imaging signal. In the normal observation mode, a blue image corresponding to the blue imaging signal, a green image corresponding to the green imaging signal, a red image corresponding to the red imaging signal are produced. In the special observation mode, first to fourth narrow band images corresponding to the first to fourth narrow band imaging signals, respectively, are produced. These images produced by the DSP 55 are stored to the frame memory 56.

The observation image generator 57 includes a normal image generator 60 and a special image generator 61. The normal image generator 60 produces a normal image in which the blue image is assigned to a B channel of a display, the green image is assigned to a G channel, and the red image is assigned to a red channel. The display control circuit 58 displays the produced normal image on the monitor 14.

The special image generator 61 includes a blood vessel enhancing unit 62, a positioning unit 63, a blood vessel enhanced image generating unit 64, and an oxygen saturation image generating unit 65. The blood vessel enhancing unit 62 enhances a blood vessel portion in the first to fourth narrow band images by a frequency filtering process. In enhancing the blood vessel portion, a superficial blood vessel is enhanced in the first and second narrow band images, both the superficial blood vessel and a middle-layer blood vessel are enhanced in the third narrow band image, and the middle-layer blood vessel is enhanced in the fourth narrow band image, in consideration of the difference in a hemoglobin light absorption property and a light scattering property of digestive mucosa among the first to fourth narrow band light used for obtaining the first to fourth narrow band images. These enhanced first to fourth narrow band images in which the predetermined blood vessels are enhanced are stored to the frame memory 56.

Note that, for example, a predetermined two dimensional filter is used for blood vessel enhancement of each layer. To produce the two dimensional filter, a frequency corresponding to a blood vessel of each layer in an image is first obtained based on the assumption of the distance between the distal portion 16a of the electronic endoscope 11 and an observation area and a magnification thereof. Next, a filter that enhances only that frequency band is designed in frequency space, and the filter is subjected to Fourier transform so as to correspond to real space. Here, it is required to adjust the property of the two dimensional filter in the frequency space such that the size of the two dimensional filter is set within a realistic size of the order of 5x5, for example.

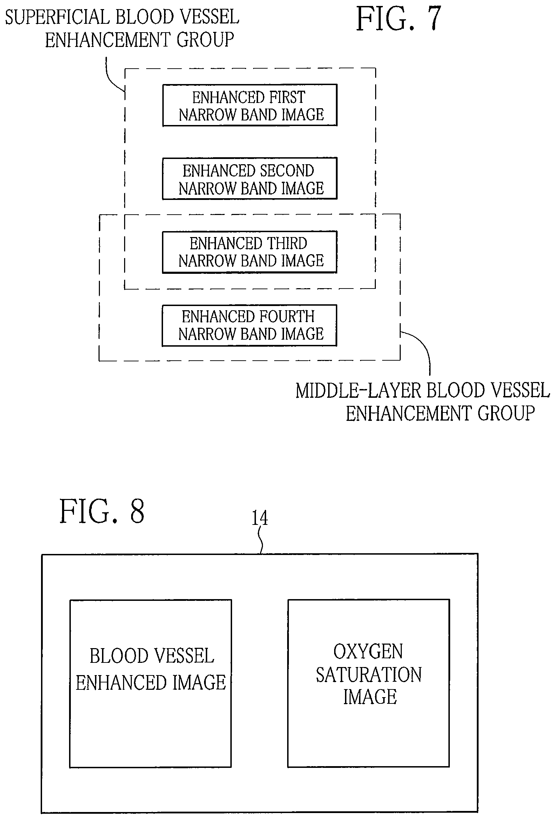

The positioning unit 63 performs positioning among the enhanced first to fourth narrow band images based on the enhanced first to fourth narrow band images. As shown in Fig. 7, the positioning is performed among the enhanced first to third narrow band images (superficial blood vessel enhancement group) in which the superficial blood vessel is enhanced, and between the enhanced third and fourth narrow band images (middle-layer blood vessel enhancement group) in which the middle-layer blood vessel is enhanced. Since the positioning is performed among images that are grouped in accordance with the type of an enhanced blood vessel, the precision of the positioning can be improved. The enhanced first to fourth narrow band images after the positioning are stored to the frame memory 56.

As a method for the positioning among the enhanced first to third narrow band images, the enhanced first narrow band image is shifted up and down and right and left by a few pixels, and a difference from the enhanced second narrow band image is obtained. By repeating this step for a plurality of times, a shift amount that minimizes a sum of an absolute value of a differential signal of each pixel is obtained. Then, the enhanced first narrow band image is shifted by this shift amount. Thus, the positioning of the enhanced first narrow band image is completed. Also as for the enhanced third narrow band image, the same procedure as that of the enhanced first narrow band image is performed. Note that, the positioning between the enhanced third narrow band image and the enhanced fourth narrow band image is performed in a like manner.

The blood vessel enhanced image generating unit 64 produces a blood vessel enhanced image by assigning the enhanced and positioned first narrow band image to the B and G channels for display and assigning the enhanced and positioned fourth narrow band image to the R channel for display. The oxygen saturation image generating unit 65 produces an oxygen saturation image by assigning the enhanced and positioned third narrow band image to the B channel for display, and assigning the enhanced and positioned fourth narrow band image to the G channel for display, and assigning the enhanced and positioned second narrow band image to the R channel for display. The display control circuit 58 displays the produced blood vessel enhanced image and the oxygen saturation image side by side on the monitor 14 as shown in Fig. 8. Displaying the blood vessel enhanced image and the oxygen saturation image side by side, as described above, facilitates easily distinguishing whether there is a cancer or not and making a detailed cancer diagnosis such as the stage of the cancer.

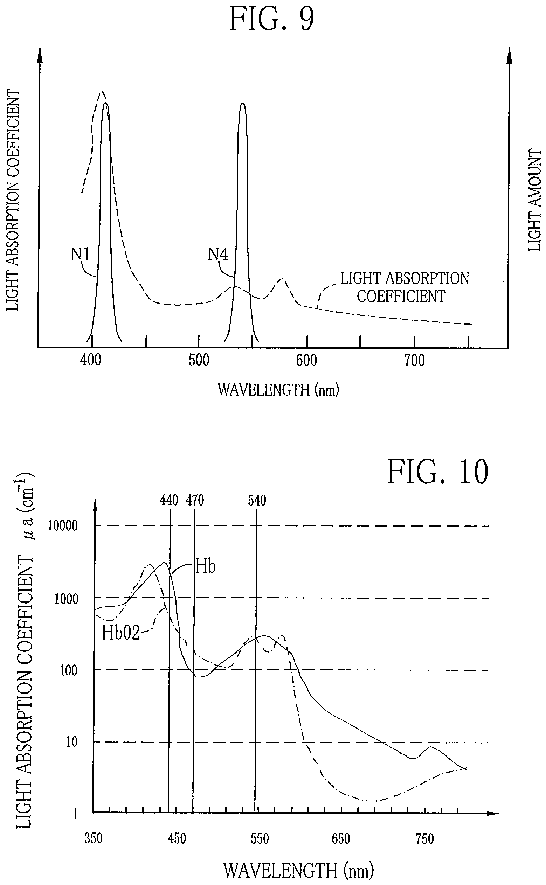

In the blood vessel enhanced image displayed on the monitor 14, the superficial blood vessel is enhanced by the first narrow band light N1 penetrating to the depth of the superficial blood vessel, and the middle-layer blood vessel is enhanced by the fourth narrow band light N4 penetrating to the depth of the middle-layer blood vessel. This is because, as shown in Fig. 9, the light absorption property of hemoglobin in blood has a peak at the wavelength band of the first narrow band light N1 used for producing the enhanced first narrow band image in the blue region, and has a peak at the wavelength band of the fourth narrow band light N4 used for producing the enhanced fourth narrow band image in the green region.

In the oxygen saturation image shown on the monitor 14, a portion having a high oxygen saturation level is colored red rather than blue, and a portion having a low oxygen saturation level is colored blue rather than red. This is because, as shown in Fig. 10, the magnitude relation of the light absorption coefficient of oxyhemoglobin HbO2 and deoxyhemoglobin Hb is reversed between the second narrow band light N2 used for producing the enhanced second narrow band image and the third narrow band light N3 used for producing the enhanced third narrow band image, and the fourth narrow band light N4 used for producing the enhanced fourth narrow band image is equal in the light absorption coefficient of oxyhemoglobin HbO2 and deoxyhemoglobin Hb.

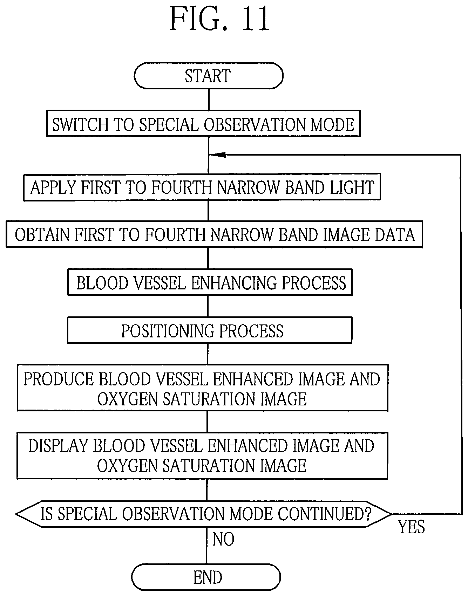

The operation of the present invention will be described in accordance with a flowchart of Fig. 11. First, the mode switch SW 20 is operated to establish the special observation mode. Accordingly, the rotary filter 32 for special observation is set in the optical path of the broad band light source 30. Rotating the rotary filter 32 for special observation sequentially applies the first to fourth narrow band light N1 to N4 to the observation object.

The CCD 44 provided in the electronic endoscope 11 images the observation obj ect irradiated with the first narrow band light N1, and output the first narrow band imaging signal. In a like manner, the CCD 44 images the observation object irradiated with the second narrow band light N2 and outputs the second narrow band imaging signal. The CCD 44 images the observation object irradiated with the third narrow band light N3 and outputs the third narrow band imaging signal. The CCD 44 images the observation object irradiated with the fourth narrow band light N4 and outputs the fourth narrow band imaging signal.

The DSP 55 provided in the processor device 12 applies various types of processes to the obtained first to fourth narrow band imaging signals. The processed first to fourth narrow band imaging signals are stored to the frame memory 56 as the first to fourth narrow band images. After that, the blood vessel enhancing unit 62 applies the blood vessel enhancing process to the first to fourth narrow band images. Since the first to third narrow band images are produced using the first to third narrow band light N1 to N3 in the blue region that penetrates to the depth of the superficial blood vessel, the frequency filtering process is applied thereto to enhance the superficial blood vessel. On the other hand, since the third and fourth narrow band images are produced using the third and fourth narrow band light N3 and N4 in the blue to green region that penetrates to the depth of the middle-layer blood vessel, the filtering process for enhancing the middle-layer blood vessel is applied thereto. Therefore, the enhanced first to fourth narrow band images are obtained.

Next, the positioning unit 63 performs positioning of the object image among the enhanced first to fourth narrow band images. The positioning unit 63 shifts a narrow band image to be positioned up and down and right and left by a few pixels, and calculates a difference from a narrow band image to be a reference . By repeating this step for a plurality of times, a shift amount that minimizes an absolute value of the difference is obtained. Then, the narrow band image to be positioned is shifted by this shift amount. Thus, the positioning of the narrow band image is completed. The enhanced first to fourth narrow band images after the positioning are stored to the frame memory 56 again.

Then, the blood vessel enhanced image is produced based on the enhanced and positioned first to fourth narrow band images, and the oxygen saturation image is produced based on the enhanced and positioned second to fourth narrow band images . The produced blood vessel enhanced image and oxygen saturation image are displayed side by side on the monitor 14. The operation flow described above is repeated during the special observation mode.

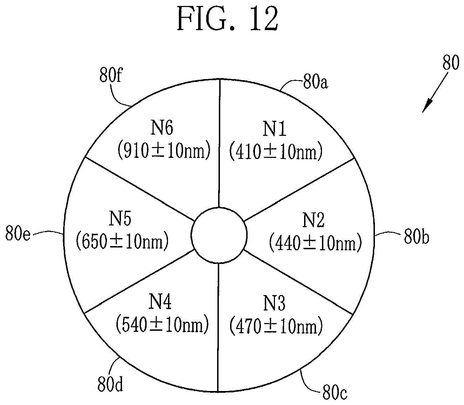

Note that, the rotary filter 32 for special observation, which sequentially transmits the first to fourth narrow band light N1 to N4, is used in the above first embodiment. Instead of this, as shown in Fig. 12, a rotary filter 80 for special observation may be used, which is provided with first to fourth narrow band light transmitting regions 80a to 80d similar to the first to fourth narrow band light transmitting regions 32a to 32d of the rotary filter 32 for special observation, a fifth narrow band light transmitting region 80e for transmitting fifth narrow band light N5 having a wavelength band of 650±10 nm out of the broad band light BB, and a sixth narrow band light transmitting region 80f for transmitting sixth narrow band light N6 having a wavelength band of 910±10 nm out of the broad band light BB. Using the fifth and sixth narrow band light N5 and N6 like this allows imaging the oxygen saturation level of the middle-layer blood vessel with high precision.

The blood vessel enhancing unit 62 applies the blood vessel enhancing process to fifth and sixth narrow band images obtained using the fifth and sixth narrow band light N5 and N6, as with the other images, to produce fifth and sixth narrow band images. Since the fifth and sixth narrow band light N5 and N6 penetrates to the depth of a deep-layer blood vessel, the fifth and sixth narrow band images are preferably subjected to a deep-layer blood vessel enhancing process. In addition to this, the fifth and sixth narrow band images are preferably subjected to the middle-layer blood vessel enhancing process for the purpose of facilitating positioning with the fourth narrow band image. After that, the positioning between the enhanced fifth and sixth narrow band images and the other enhanced first to fourth narrow band images is performed. A positioning method is the same as that of the above first embodiment.

To produce the oxygen saturation image, as with the above first embodiment, a first oxygen saturation image is produced based on the second to fourth narrow band light N2 to N4, and a second oxygen saturation image is produced based on fourth to sixth narrow band light N4 to N6. The first oxygen saturation image is assigned to the RGB channels for display in the same manner as the above first embodiment. As for the second oxygen saturation image, on the other hand, the enhanced and positioned sixth narrow band image is assigned to the B channel for display, the enhanced and positioned fourth narrow band image is assigned to the G channel for display, and the enhanced and positioned fifth narrow band image is assigned to the R channel for display.

The produced blood vessel enhanced image, the first oxygen saturation image, and the second oxygen saturation image are displayed side by side on the monitor 14. The distribution of the oxygen saturation level of the superficial blood vessel is precisely reflected in the first oxygen saturation image. The distribution of the oxygen saturation level of the middle to deep-layer blood vessels is precisely reflected in the second oxygen saturation image. Thus, using the first and second oxygen saturation images makes it possible to observe an oxygen saturation state of living body tissue in a depth direction.

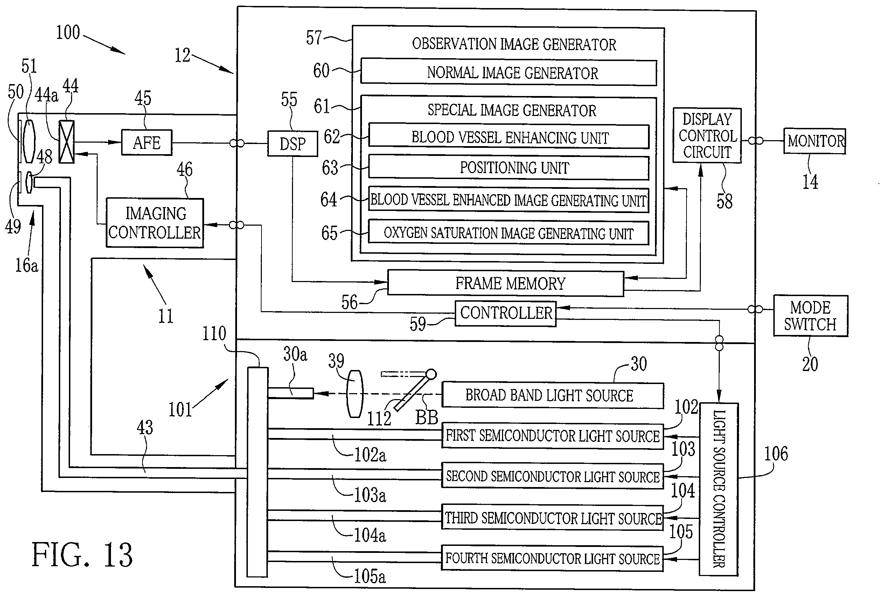

As shown in Fig. 13, an endoscope system 100 according to a second embodiment adopts in a light source device 101 a method using a semiconductor light source that is switchable between turn-on (ON) and turn-off (OFF) at high speed, instead of a rotary filter method as described in the first embodiment. As the semiconductor light source, a laser, a LED (light emitting diode), or the like is usable. Note that, the components other than the light source device are identical to those of the first embodiment, so the description thereof will be omitted.

The light source device 101 is provided with a first semiconductor light source 102 for emitting first narrow band light N1 having a wavelength band of 410±10 nm, a second semiconductor light source 103 for emitting second narrow band light N2 having a wavelength band of 440±10 nm, a third semiconductor light source 104 for emitting third narrow band light N3 having a wavelength band of 470±10 nm, and a fourth semiconductor light source 105 for emitting fourth narrow band light N4 having a wavelength band of 540± nm, and a light source controller 106 for controlling the operation of the first to fourth semiconductor light sources 102 to 105, in addition to the broad band light source 30, which is identical to that of the first embodiment.

The broad band light BB from the broad band light source 30 is incident upon an optical fiber 30a through the condenser lens 39. On the other hand, the first to fourth narrow band light N1 to N4 from the first to fourth semiconductor light sources 102 to 105 is incident upon optical fibers 102a to 105a, respectively. These optical fibers 30a and 102a to 105a are coupled to the light guide 43 via a coupler 110. Thus, the light led through each of the optical fibers 30a and 102a to 105a enters the light guide 43 through the coupler 110. The light source device 101 has a shutter 112, which is shiftable between a position inserted into the optical path of the broad band light source 30 to block the entrance of the broad band light BB to the optical fiber 30a and a position retracted from the optical path to allow the entrance of the broad band light BB to the optical fiber 30a. Note that, it is unnecessary to provide the shutter 112 if the broad band light source 30 uses a light source that can instantaneously switchable between ON and OFF such as a white LED.

In the second embodiment, the shutter 112 is set in the retracted position in the normal observation mode. At this time, the first to fourth semiconductor light sources 102 to 105 are always turned off. Thus, only the broad band light BB is applied to the observation object, and a normal image is produced based on the broad band light BB. In the special observation mode, on the other hand, while the shutter 112 is set in the retracted position, the first to fourth semiconductor light sources 102 to 105 are sequentially turned on. Thus, the first to fourth narrow band light N1 to N4 is sequentially applied to the observation object, while the broad band light BB is blocked.

Note that, the oxygen saturation level is imaged in the above first and second embodiments, but an oxyhemoglobin index calculated by "blood volume (the sum of oxyhemoglobin and deoxyhemoglobin) × oxygen saturation level (%)" or a deoxyhemoglobin index calculated by "blood volume × (100-oxygen saturation level) (%) " may be calculated instead of or in addition to this.

Note that, in the above first and second embodiments, the blood vessel enhancing process is applied in order to increase precision in the positioning of a blood vessel between images. However, if the positioning is performed based on various types of structure, an outline, and the like of the observation object such as an edge of mucosa, other than the blood vessel, an enhancing process may be applied to the various types of structure and the outline.

## Claims

1. An endoscope system (10) comprising:
a lighting section (13) for sequentially applying to an observation object, first illumination light in a wavelength band having a high light absorption coefficient of hemoglobin, out of light in a wavelength band penetrating to a depth of a specific layer in living body tissue, and second illumination light in a wavelength band that is different from said wavelength band of said first illumination light and in which oxyhemoglobin and deoxyhemoglobin have different light absorption coefficients;
an image signal obtaining section (11, 12) for obtaining a first image signal by imaging said observation object under irradiation with said first illumination light and obtaining a second image signal by imaging said observation object under irradiation with said second illumination light;
**characterized by**
an image generating section (57) adapted to producing a first object image based on only said first image signal and producing a second object image based on said second image signal; and
a display section (14) for displaying said first and second object images.

2. The endoscope system (10) according to claim 1, **characterized in that** said image signal obtaining section (12) includes a positioning unit (63) for performing positioning of an object image between said first and second image signals.

3. The endoscope system (10) according to claim 2, **characterized in that**
said image signal obtaining section (12) further includes a structure enhancing unit (62) for applying a structure enhancing process to said first and second image signals to enhance a structure of said observation object; and
said positioning unit (63) performs positioning of said object image between said first and second image signals after being subjected to said structure enhancing process.

4. The endoscope system (10) according to claim 3, **characterized in that** said structure of said observation object includes a blood vessel structure.

5. The endoscope system (10) according to one of claims 1 to 4, **characterized in that** said lighting section (13) includes:
a broad band light source (30) for emitting broad band light in a broad wavelength band; and
a rotary filter (32) for sequentially transmitting said first and second illumination light out of said broad band light.

6. The endoscope system (100) according to one of claims 1 to 4 , **characterized in that** said lighting section (101) includes a plurality of semiconductor light sources (102-105) for emitting said first and second illumination light.

7. The endoscope system (10) according to one of claims 1 to 4, **characterized in that**
said first illumination light contains at least blue narrow band light having a wavelength band in a blue region and green narrow band light having a wavelength band in a green region, and said second illumination light contains at least narrow band light having two non-isosbestic wavelengths at which a magnitude relation of a light absorption coefficient between said oxyhemoglobin and said deoxyhemoglobin differs from each other;
said first image signal includes a blue narrow band signal obtained by imaging said observation object under irradiation with said blue narrow band light and a green narrow band signal obtained by imaging said observation object under irradiation with said green narrow band light;
said second image signal includes a first non-isosbestic wavelength narrow band signal obtained by imaging said observation object under irradiation with one of said narrow band light having said two non-isosbestic wavelengths and a second non-isosbestic wavelength narrow band signal obtained by imaging said observation object under irradiation with the other of said narrow band light;
said first object image is a blood vessel enhanced image produced based on only said blue narrow band signal and said green narrow band signal; and
said second object image is an oxygen saturation image produced based on said first non-isosbestic wavelength narrow band signal and said second non-isosbestic wavelength narrow band signal.

8. The endoscope system (10) according to claim 7, **characterized in that**
said blue narrow band light is in a wavelength band of 410±10 nm;
said green narrow band light is in a wavelength band of 540±10 nm; and
said narrow band light having said two non-isosbestic wavelengths is in wavelength bands of 440±10 nm and 470±10 nm.

9. The endoscope system (10) according to claim 8, **characterized in that** said second illumination light further includes narrow band light in a wavelength band of 650±10 nm and narrow band light in a wavelength band of 910±10 nm, as said narrow band light having said non-isosbestic wavelengths.

## Patentansprüche

1. Endoskopsystem (10) umfassend:
einen Beleuchtungsabschnitt (13) zum sequenziellen Aufbringen von erstem Beleuchtungslicht eines Wellenlängenbands mit einem hohen Lichtabsorptionskoeffizienten von Hämoglobin im Rahmen von Licht eines Wellenlängenbands, das in eine Tiefe einer spezifischen Schicht in lebendem Körpergewebe eindringt, und von zweitem Beleuchtungslicht in einem Wellenlängenband verschieden von dem Wellenlängenband des ersten Beleuchtungslichts, und in welchem Oxyhämoglobin und Deoxyhämoglobin unterschiedliche Lichtabsorptionskoeffizienten besitzen, auf ein Betrachtungsobjekt;
einen Bildsignal-Gewinnungsabschnitt (11, 12) zum Gewinnen eines ersten Bildsignals durch Abbilden des Betrachtungsobjekts unter Bestrahlung mit dem ersten Beleuchtungslicht und zum Gewinnen eines zweiten Bildsignals durch Abbilden des Betrachtungsobjekts unter Bestrahlung mit dem zweiten Beleuchtungslicht;
**gekennzeichnet durch**
einen Bilderzeugungsabschnitt (57), ausgebildet zum Erzeugen eines ersten Objektbilds basierend auf nur dem ersten Bildsignal, und zum Erzeugen eines zweiten Objektbilds basierend auf dem zweiten Bildsignal; und
einen Anzeigeabschnitt (14) zum Anzeigen des ersten und des zweiten Objektbilds.

2. Endoskopsystem (10) nach Anspruch 1, **dadurch gekennzeichnet, dass** der Bildsignal-Gewinnungsabschnitt (12) eine Positioniereinheit (63) zum Ausführen einer Positionierung eines Objektbilds zwischen dem ersten und dem zweiten Bildsignal aufweist.

3. Endoskopsystem (10) nach Anspruch 2, **dadurch gekennzeichnet, dass**
der Bildsignal-Gewinnungsabschnitt (12) weiterhin eine Strukturverstärkungseinheit (62) aufweist zum Anwenden eines Strukturverstärkungsprozesses auf das erste und das zweite Bildsignal, um eine Struktur des Betrachtungsobjekts zu verstärken; und
wobei die Positioniereinheit (63) eine Positionierung des Objektbilds zwischen dem ersten und dem zweiten Bildsignal ausführt, nachdem diese dem Strukturverstärkungsprozess unterzogen wurden.

4. Endoskopsystem (10) nach Anspruch 3, **dadurch gekennzeichnet, dass** die Struktur des Betrachtungsobjekts eine Blutgefäßstruktur enthält.

5. Endoskopsystem (10) nach einem der Ansprüche 1 bis 4, **dadurch gekennzeichnet, dass** der Beleuchtungsabschnitt (13) enthält:
eine Breitband-Lichtquelle (30) zum Emittieren von breitbandigem Licht in einem breiten Wellenlängenband; und
ein Drehfilter (32) zum sequenziellen Durchlassen des ersten und des zweiten Beleuchtungslichts von dem breitbandigen Licht.

6. Endoskopsystem (100) nach einem der Ansprüche 1 bis 4, **dadurch gekennzeichnet, dass** der Beleuchtungsabschnitt (101) mehrere Halbleiterlichtquellen (102-105) zum Emittieren des ersten und des zweiten Beleuchtungslichts enthält.

7. Endoskopsystem (10) nach einem der Ansprüche 1 bis 4, **dadurch gekennzeichnet, dass**
das erste Beleuchtungslicht mindestens blaues schmalbandiges Licht mit einem Wellenlängenband in einer Blauzone und grünes schmalbandiges Licht mit einem Wellenlängenband in einer Grünzone enthält, und das zweite Beleuchtungslicht mindestens schmalbandiges Licht mit zwei nicht-isobestischen Wellenlängen enthält, bei denen eine Größenrelation eines Lichtabsorptionskoeffizienten zwischen dem Oxyhämoglobin und dem Deoxihämoglobin unterschiedlich sind, dass
das erste Bildsignal ein blaues schmalbandiges Bildsignal enthält, gewonnen durch Abbilden des Betrachtungsobjekts unter Bestrahlung mit dem blauen schmalbandigen Licht, und ein grünes schmalbandiges Signal enthält, gewonnen durch Abbilden des Betrachtungsobjekts unter Bestrahlung mit dem grünen schmalbandigen Licht; dass
das zweite Bildsignal ein erstes Wellenlängen-isobestisches schmalbandiges Signal enthält, gewonnen durch Abbilden des Betrachtungsobjekts unter Bestrahlung mit einem von dem schmalbandigen Licht mit zwei nicht-isobestischen Wellenlängen und einem zweiten nicht-isobestischen schmalbandigen Signal, gewonnen durch Abbilden des Betrachtungsobjekts unter Bestrahlung mit dem anderen von dem schmalbandigen Licht; dass
das erste Objektbild ein Blutgefäß-verstärktes Bild ist, erzeugt basierend auf nur dem blauen schmalbandigen Signal und dem grünen schmalbandigen Signal; und
das zweite Objektbild ein Sauerstoffsättigungsbild ist, erzeugt auf der Grundlage des ersten nicht-isobestischen schmalbandigen Signals und des zweiten nicht-isobestischen schmalbandigen Bildsignals.

8. Endoskopsystem (10) nach Anspruch 7, **dadurch gekennzeichnet, dass**
das blaue schmalbandige Licht ein Wellenlängenband von 410±10 nm ist;
das grüne schmalbandige Licht ein Wellenlängenband von 540±10 nm ist, und
das schmalbandige Licht mit den zwei nicht-isobestischen Wellenlängen in Wellenlängenbändern von 440±10 nm und 470±10 nm liegt.

9. Endoskopsystem (10) nach Anspruch 8, **dadurch gekennzeichnet, dass** das zweite Beleuchtungslicht weiterhin schmalbandiges Licht in einem Wellenlängenband von 650±10 nm und schmalbandiges Licht in einem Wellenlängenband von 910±10 nm als das schmalbandige Licht mit nicht-isobestischen Wellenlängen enthält.

## Revendications

1. Système d'endoscope (10), comprenant :
une section d'éclairage (13) pour appliquer séquentiellement, à un objet d'observation, une première lumière d'éclairage dans une bande de longueurs d'onde présentant un coefficient d'absorption de la lumière élevé pour l'hémoglobine, à partir d'une lumière dans une bande de longueurs d'onde pénétrant jusqu'à une profondeur d'une couche spécifique dans un tissu corporel vivant, et une seconde lumière d'éclairage dans une plage de longueurs d'onde différente de ladite bande de longueurs d'onde de ladite première lumière d'éclairage et, où l'oxyhémoglobine et la désoxyhémoglobine présentent des coefficients d'absorption de la lumière différents ;
une section d'obtention de signal d'image (11, 12), pour obtenir un premier signal d'image en imageant ledit objet d'observation sous une irradiation à l'aide de ladite première lumière d'éclairage et obtenir un second signal d'image en imageant ledit objet d'observation sous une irradiation avec ladite seconde lumière d'éclairage ;
**caractérisé par**
une section de génération d'image (57), apte à produire une première image d'objet sur la base uniquement dudit premier signal d'image, et produire une seconde image d'objet sur la base dudit second signal d'image, et
une section d'affichage (14) pour afficher lesdites première et seconde images d'objet.

2. Système d'endoscope (10) selon la revendication 1, **caractérisé en ce que** ladite section d'obtention de signal d'image (12) inclut une unité de positionnement (63) pour réaliser un positionnement d'une image d'objet entre lesdits premier et second signaux d'image.

3. Système d'endoscope (10) selon la revendication 2, **caractérisé en ce que**
ladite section d'obtention de signal d'image (12) inclut en outre une unité de mise en valeur de structure (62) pour appliquer un processus de mise en valeur de structure auxdits premier et second signaux d'image afin de mettre en valeur une structure dudit objet d'observation, et
ladite unité de positionnement (63) réalise un positionnement de ladite image d'objet entre lesdits premier et second signaux d'image après soumission audit processus de mise en valeur de structure.

4. Système d'endoscope (10) selon la revendication 3, **caractérisé en ce que** ladite structure dudit objet d'observation inclut une structure de vaisseau sanguin.

5. Système d'endoscope (10) selon l'une quelconque des revendications 1 à 4. **caractérisé en ce que** ladite section d'éclairage (13) inclut :
une source de lumière à large bande (30) pour émettre une lumière à large bande dans une large bande de longueurs d'onde, et
un filtre rotatif (32) pour transmettre séquentiellement lesdites première et seconde lumières d'éclairage à partir de ladite lumière à large bande.

6. Système d'endoscope (100) selon l'une quelconque des revendications 1 à 4, **caractérisé en ce que** ladite section d'éclairage (101) inclut une pluralité de sources de lumière à semi-conducteur (102-105) pour émettre des première et seconde lumières d'éclairage.

7. Système d'endoscope (10) selon l'une quelconque des revendications 1 à 4, **caractérisé en ce que**
ladite première lumière d'éclairage contient au moins une lumière à bande étroite bleue présentant une bande de longueurs d'onde dans une région bleue et une lumière à bande étroite bleue présentant une bande de longueurs d'onde dans une région verte, et ladite seconde lumière d'éclairage contient au moins une lumière à bande étroite présentant deux longueurs d'onde non isosbestiques sur lesquelles une relation de grandeur d'un coefficient d'absorption de la lumière entre ladite oxyhémoglobine et ladite désoxyhémoglobine est différente
ledit premier signal d'image inclut un signal à bande étroite bleu obtenu en imageant ledit objet d'observation sous une irradiation avec ladite lumière à bande étroite bleue et un signal à bande étroite vert obtenu en imageant ledit objet d'observation sous une irradiation avec ladite lumière à bande étroite verte ;
ledit second signal d'image inclut un premier signal à bande étroite de longueur d'onde non isosbestique obtenu en imageant ledit objet d'observation sous une irradiation avec une lumière de ladite lumière à bande étroite présentant lesdites deux longueurs d'onde non isosbestiques et un second signal à bande étroite de longueur d'onde non isosbestique obtenu en imageant ledit objet d'observation sous une irradiation avec l'autre lumière de ladite lumière à bande étroite ;
ladite première image d'objet est une image de mise en valeur de vaisseau sanguin produite sur la base uniquement dudit signal à bande étroite bleu et dudit signal à bande étroite vert, et
ladite seconde image d'objet est une image de saturation d'oxygène produite sur la base dudit premier signal à bande étroite de longueur d'onde non isosbestique et dudit second signal à bande étroite de longueur d'onde non isosbestique.

8. Système d'endoscope (10) selon la revendication 7, **caractérisé en ce que**
ladite lumière à bande étroite bleue est une bande de longueurs d'onde de 410 ± 10nm ;
ladite lumière à bande étroite verte est une bande de longueurs d'onde de 540 ±10 nm, et
ladite lumière à bande étroite présentant lesdites deux longueurs d'onde non isosbestiques est comprise dans des bandes de longueurs d'onde de 440 ± 10 nm et 470 ± 10 nm.

9. Système d'endoscope (10) selon la revendication 8, **caractérisé en ce que** ladite seconde lumière d'éclairage inclut en outre une lumière à bande étroite dans une bande de longueurs d'onde de 650 ±10 nm et une lumière à bande étroite dans une bande de longueurs d'onde de 910 ±10 nm, comme dite lumière à bande étroite présentant lesdites longueurs d'onde non isosbestiques.
